Europäisches Patentamt

(19) European Patent Office

Office européen des brevets

(11) Publication number: **0 333 676**
**A2**

(12) **EUROPEAN PATENT APPLICATION**

(21) Application number: **89830120.5**

(22) Date of filing: **16.03.89**

(51) Int. Cl.⁴: **A 61 K 31/495**

(30) Priority: **18.03.88 IT 4774988**

(43) Date of publication of application:
**20.09.89 Bulletin 89/38**

(84) Designated Contracting States:
**AT BE CH DE FR GB GR LI LU NL SE**

(71) Applicant: **MALESCI ISTITUTO FARMACOBIOLOGICO S.p.A.**
**Via Nicola Porpora 22/24**
**I-50144 Firenze (IT)**

(72) Inventor: **Manzini, Stefano**
**Via della Mattonaia No. 25**
**Firenze (IT)**

**Fedi, Mauro**
**Via Campanella No. 61**
**I-50019 Sesto Fiorentino (FI) (IT)**

(74) Representative: **Taliercio, Antonio et al**
**ING. BARZANO' & ZANARDO ROMA S.p.A. Via Piemonte, 26**
**I-00187 Roma (IT)**

(54) Use of 1-(2-ethoxy-2-(3'-pyridyl)ethyl)-4-(2'methoxy-phenyl)piperazine, for the preparation of pharmaceutical compositions useful for the treatment of lower urinary tract disfunctions and benign prostatic hypertrophy.

㊼ Use of 1-(2-ethoxy-2-(3'-pyridyl)ethyl)-4-(2'-methoxy-phenyl)piperazine, or its enantiomers or pharmaceutically acceptable addition salts thereof, for the preparation of pharmaceutical compositions useful for the treatment of lower urinary tract disfunctions and benign prostatic hypertrophy.

EP 0 333 676 A2

## Description

### Use of 1-(2-ethoxy-2-(3'-pyridyl)ethyl)-4-(2'-methoxy-phenyl)piperazine, for the preparation of pharmaceutical compositions useful for the treatment of lower urinary tract disfunctions and benign prostatic hypertrophy

The invention relates to the use of racemic 1-(2-ethoxy-2-(3'-pyridyl) ethyl)-4-(2'-methoxy-phenyl)piperazine, and its enantiomers and pharmaceutically acceptable acid addition salts thereof for the treatment of lower urinary tract disfunctions (detrusor hyperreflexia, acute urinary ritention of various genesis, etc.) and benign prostatic hypertrophy.

1-(2-ethoxy-2-(3'-pyridyl)ethyl )-4-(2'-methoxy-phenyl )piperazine is a known potent alpha-adrenoceptor antagonist developed in Malesci laboratories (Giannini M. - It. Pat. 1056055) as antihypertensive agent.

Recent findings indicate its strong potency in blocking postjunctional but not prejunctional alpha-adrenergic responses (Castellucci A. et al. - Arzneim. Forsch. (Drug Res) 34 (1), 150; 1984; Castellucci A. et al. - ibidem 35 (1), 107; 1985) suggesting selectivity towards alpha$_1$-adrenoceptor.

Very recently it has been shown that stimulation of alpha-adrenoceptor, mainly of the alpha$_1$-subtype, produces marked contraction of urethral (Honda et al. - Naunyn Schmied. Arch. Pharmacol. 330, 16; 1985) and prostatic (Hieble et al. - Eur. J. Pharmacol. 107, 111; 1985) smooth muscle. The overexcitation of these alpha-adrenoceptors produces urinary retention and this might be relevant in the etiology of the lower urinary tract disfunctions and particularly of benign prostatic hypertrophy.

In view of the above, it appeared to the Applicant worthwhile to investigate the effect of the compound of present invention in the treatment of the lower urinary tract disfunctions and of benign prostatic hypertrophy; the possibility of its use in the preparation of pharmaceutical compositions for treatment of such diseases and finally, the therapeutical methods of treatment.

The effect of the compound of the present invention has been, therefore, investigated on the isolated urethra of rat and in animal models characterized by urethral outflow obstruction.

It was quite surprising to discover that the compound of present invention in the proposed "in vivo" tests, proved to be effective, in improving voiding efficiency at doses devoid of hypotensive effect in normotensive animals. Thus the compound of invention may be used conveniently, without undesiderable side effects, for the treatment of lower urinary tract disfunctions.

It is therefore specific object of the present invention the use of racemic 1-(2-ethoxy-2-(3'-pyridyl)ethyl)-4-(2'-methoxy-phenyl)piperazine, or its enantiomers or pharmaceutically acceptable salts thereof, for the preparation of pharmaceutical compositions useful for the treatment of lower urinary tract disfunctions and benign prostatic hypertrophy.

Pharmaceutical compositions prepared, according to the present invention, for the use abovementioned, can be administered orally, rectally, topically, by inhalation and by parenteral (intramuscolar, intravenous, subcutaneous) injection, as free base or acid addition salt thereof (e.g. hydrochloride, sulfate, maleate, acetate, citrate, etc.).

It is appropriate to administer 0,1-10 mg daily doses of the compound in the case of parenteral administration, or 1-20 mg in the case of oral or rectal administration.

The compound of this invention may be formulated into ordinary dosage forms such as, for example, tablets, capsules, pills, solutions, suppositories, ointments, etc. and also into retard or sustained-release forms and the medicament can be prepared by conventional methods using usual medical excipients.

The following Examples illustrate how the compounds of the invention can be incorporated in pharmaceutical compositions, but they are not intended to limit it.

### Example 1.

Tablets

| | |
|---|---|
| 1-(2-ethoxy-2-(3'pyridil)ethyl)-4-(2'-methoxy-phenyl)-piperazine hydrochloride | 1.0 mg |
| Cellulose | 15.5 mg |
| Dibasic Calcium Phosphate dihydrate | 37.0 mg |
| Magnesium Stearate | 1.5 mg |

### Example 2.

Suppositories

| | |
|---|---|
| 1-(2-ethoxy-2-(3'pyridil)ethyl)-4-(2'-methoxy-phenyl)-piperazine hydrochloride | 1.0 mg |
| Semi-synthetic Glycerides | ad 2000 mg |

### Example 3.

Injection solutions

| | |
|---|---|
| 1-(2-ethoxy-2-(3'pyridil)ethyl)-4-(2'-methoxy-phenyl)-piperazine hydrochloride | 0.2 mg |
| Physiological solution | ad 1.0 ml |

### IN VITRO STUDIES

In the "in vitro" studies, male albino rats, Wistar Nossan strain, weighing 300-350 g were used. Animals were sacrificed by cervical dislocation and the proximal urethra was quickly excised, cleaned of connective tissues and then suspended in an organ bath and tied to an isometric force transducer, under resting tension of 0.5 g. Experiments were performed at 37°C and the bathing medium (gassed with 95% $O_2$ and 5% $CO_2$) had the following composition (mM): NaCl 119, KCl 4.7, $MgSO_4$ 1.5, $KH_2PO$ 41.2, $CaCl_2$ 2.5, $NaHCO_3$ 25 and glucose 11. Experiments were carried out after 60 min equilibration period.

In a first group of experiments a cumulative concentration response curve to alpha$_1$-adrenoceptor selective agonist, phenylephrine, was obtained in control conditions or in presence of the compound of invention (1 nM). The compound of invention (1 nM) produced a rightward parallel displacement of concentration response curve to phenylephrine with a $pA_2$ of $9.18 \pm 0.12$. In another set of experiments urethral contractions were obtained by means of field stimulation (20 Hz, 0.5 msec, maximal voltage; trains of 5 sec every 4 min). These contractions (obtained in a medium additionated with atropine 1 uM) were TTX-sensitive and probably due to stimulation of adrenergic fibers. Compound of invention in the range 1 pM-1 nM, elicited a potent and concentration-dependent inhibitory effect, its $EC_{50}$ being 0.13 nM (0.09-0.18).

## IN VIVO STUDIES

In the "vivo" studies two experimental models characterized by urethral outflow obstruction were used:
a) rat chronically treated with testosterone and
b) rat acutely treated with hexamethonium.

### a) Rat treated with testosterone

Male albino rats, Wistar strain, weighting 300-350 g, were treated for 2 weeks with testosterone propionate at the dose of 3 mg/Kg s.c.. Afterwards animals were anaesthetized with urethane 40% s.c. (0.3 ml/hg) and then transvescical cystometrogram were perfused according to the method of Maggi et al. (J. Pharmacol. Meth. 15, 157; 1986). Testosterone-treated animals were characterized by the appearance of frequent, scarcely effective, micturition complexes and by a very high residual volume (about double than controls). In these conditions treatment with compound of invention (0.1-3 ug/Kg i.v.) elicited a clear, objective and prompt beneficial action on the voiding efficiency of these animals. It is important to underline that in urethane anaesthetized normotensive rats, i.v. administration of compound of invention exerts an hypotensive effect in the range 3-100 ug/kg.

### b) Rat treated with hexamethonium

Urethane-anaesthetized male albino rats, Wistar strain weighing 300-350 g, were used.
Administration of hexamethonium (40 mg/Kg i.v.) 5 minutes before start of transvescical cystometrogram produced in about 75% of animals an overflow incontinence coupled to high values of intravescical pressure. In animals pretreated with the compound of invention (at doses lower than 3 ug/Kg i.v. hexamethonium administration never produced overflow incontinence and in addition significantly lower intravescical pressure values were measured.

## Claims

1. Use of racemic 1-(2-ethoxy-2-(3'-pyridyl)ethyl)-4-(2'-methoxy-phenyl)piperazine, or its enantiomers, or pharmaceutically acceptable addition salts thereof, for the preparation of pharmaceutical compositions useful for the treatment of lower urinary tract disfunctions and benign prostatic hypertrophy.

2. Use of racemic 1-(2-ethoxy-2-(3'-pyridyl)ethyl)-4-(2'-methoxy-phenyl)piperazine, or its enantiomers, or pharmaceutically acceptable addition salts thereof, for the preparation of pharmaceutical compositions according to claim 1, to be administered orally, rectally, topically, by inhalation or by parenteral (intramuscolar, intravenous, subcutaneous) injection.

3. Use of racemic 1-(2-ethoxy-2-(3'-pyridyl)ethyl)-4-(2'-methoxy-phenyl)piperazine, or its enantiomers, or pharmaceutically acceptable addition salts thereof, for the preparation of pharmaceutical compositions to be administered by parenteral (intravenous, intramuscolar, subcutaneous) injection according to claim 2, said compositions being prepared so that 0,1-10 mg daily doses of said compound are administered.

4. Use of racemic 1-(2-ethoxy-2-(3'-pyridyl)ethyl)-4-(2'-methoxy-phenyl)piperazine, or its enantiomers, or pharmaceutically acceptable addition salts thereof, for the preparation of pharmaceutical compositions to be administered orally or rectally, or topically according to claim 2, said compositions being prepared so that 1-20 mg daily doses of said compound are administered.

5. Use of racemic 1-(2-ethoxy-2-(3'-pyridyl)ethyl)-4-(2'-methoxy-phenyl)piperazine, or its enantiomers, or pharmaceutically acceptable addition salts thereof, for the preparation of pharmaceutical compositions useful for the treatment of lower urinary tract disfunctions and benign prostatic hypertrophy, according to claim 1, wherein said compositions are formulated in dosage forms such as tablets, capsules, pills, solutions, suppositories, ointments, or into retard or sustained release forms.